# EUROPEAN PATENT APPLICATION

(11) **EP 4 541 892 A1**
(43) Date of publication of application: **23.04.2025**
(21) Application number: 23943957.3
(22) Date of filing: 19.10.2023
(51) Int. Cl.: C12N 15/11, C12Q 1/6895, A01H 1/04, A01H 5/00, A01H 6/46

(54) **MAIZE TRANSGENIC EVENT QY2569-42 AND METHOD FOR DETECTING SAME**

(30) Priority: 28.08.2023 CN 202311093966
(71) Applicant: Qingdao Kingagroot Seed Science Co., Ltd., Qingdao, Shandong 266000 (CN)
(72) Inventor: CHEN, Bo, Shandong 266000 (CN); LI, Huarong, Shandong 266000 (CN); WU, Yongchun, Shandong 266000 (CN); LIAN, Lei, Shandong 266000 (CN); DING, Dehui, Shandong 266000 (CN)
(74) Representative: De Vries & Metman
(86) International application number: PCT/CN2023/125356
(87) International publication number: WO 2025/043835

(57) **Abstract**

The present invention relates to a transgenic corn event, QY2569-42, and further relates to a nucleic acid sequence for detecting a corn plant QY2569-42 and a detection method thereof. The nucleic acid sequence of the corn plant comprises SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:10, SEQ ID NO:11 and/or SEQ ID NO:12, or a nucleic acid sequence complementary thereto. The corn plant QY2569-42 of the present invention has good resistance against lepidopteran insects and good tolerance to glufosinate herbicide without impacts on yield. In addition, the detection method can accurately and rapidly identify whether a biological sample comprises DNA molecules of transgenic corn event QY2569-42.

## Description

### Technical Field

The present invention relates to a transgenic corn event, QY2569-42, and further relates to a nucleic acid sequence for detecting a corn plant QY2569-42 and a detection method thereof.

### Background Art

Agricultural pests are the number one important factor affecting crop production. At present, chemical pesticides are still the main role in pest control, playing a crucial part in maintaining the stable development of agricultural production. Pesticides act in a non-specific poisoning manner, which has poisonous effects to beneficial insects and birds while killing target insect pests. Long-term use will cause serious pollution to the natural environment, destroy the ecological balance, and due to its enrichment in the food chain, it will directly pose a threat to the safety of humans and animals. Weeds are the second most important factor affecting crop production. They compete with crops for water resources, fertilizers, light sources, etc., resulting in reduced crop yields and lower quality of agricultural products in the meantime. The traditional manual weeding approach is time-consuming, laborious and inefficient, and mechanical weeding is expensive, while chemical weeding has high technical requirements and is easy to cause phytotoxicity to plants, affecting the growth and development of the crops themselves. Meanwhile, chemical agents may also affect sensitive crops nearby.

Corn (*Zea mays* L.) is a major food crop in many regions of the world. Presently, transgenic breeding technology has been widely applied to corn to improve its agronomic traits and quality. Insect resistance is an important agronomic trait in corn production, especially resistance to lepidopteran insects such as *Ostrinia furnacalis, Spodoptera frugiperda, Helicoverpa armigera, Mythimna separata,* etc. The resistance of corn to lepidopteran insects may be acquired by expressing lepidopteran resistance genes (e.g., *mvip3Aa20, cry1Ab*) in corn plants through transgenic methods. Another important agronomic trait is herbicide tolerance, for example, tolerance to glufosinate herbicide. The tolerance of corn to glufosinate herbicide may be acquired by expressing glufosinate herbicide tolerance genes (e.g., *pat*) in corn plants through transgenic methods.

An insect resistance trait and/or herbicide tolerance trait may be used alone or in combination with other traits (e.g., tolerance to other herbicides or resistance to other insect pests or pathogens). A combination of traits can be achieved by breeding each individual trait together. However, breeding individual traits in corn together and maintaining the combination during breeding with multiple elite germplasm is a time-consuming and expensive process. A combination of traits may also be achieved by combining multiple insect resistance traits and/or herbicide tolerance traits at one location or locus in the corn genome, which simplifies the breeding process and is an economical and effective transgenic breeding measure.

The expression of a transgene in a plant is influenced by multiple factors such as the site of insertion into the genome of the recipient plant, the elements used in the expression cassette, and the interactions between the elements. This is more complex for a transgene insertion comprising two or more expression cassettes, with each having a transgene conferring a separate trait, which is also known as a multi-gene transgenic event. The expression level and expression pattern of an exogenous gene may vary significantly from event to event. Therefore, it is first necessary to select the optimal expression cassette for a single trait, build a construct, and obtain a large number of transgenic events. And through rigorous molecular characterization, greenhouse testing as well as multi-year field trials in multiple locations and under various conditions, extensive agronomic, phenotypic and molecular data are collected to screen out superior events with expected transgene expression levels and expression patterns for commercialization purposes from the transgenic events.

### Description of the Invention

The purpose of the present invention is to provide a transgenic corn event QY2569-42 as well as a nucleic acid sequence for detecting transgenic corn event QY2569-42 and a detection method.

The technical scheme adopted in the present invention is as follows:
The present invention provides a nucleic acid sequence, which comprises:
(a) SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 10, SEQ ID NO: 11 and/or SEQ ID NO: 12; or
(b) a nucleic acid sequence complementary to (a)

The nucleic acid sequence is derived from transgenic corn event QY2569-42.

In one specific embodiment, the nucleic acid sequence is an amplicon diagnostic for the presence of corn event QY2569-42.

The present invention also provides a DNA construct comprising three expression cassettes, wherein,
a) the first expression cassette comprises in operable linkage an OsUbi2 promoter with a nucleic acid sequence as shown in SEQ ID NO: 29, a *mVip3Aa20* gene coding region with a nucleic acid sequence as shown in SEQ ID NO: 30, a chloroplast leading peptide CTP-TS-SSU with a nucleic acid sequence as shown in SEQ ID NO: 31, a T-Ara5 terminator with a nucleic acid sequence as shown in SEQ ID NO: 32 that terminates the expression of the gene;
b) the second expression cassette comprises in operable linkage a P-FMV promoter with a nucleic acid sequence as shown in SEQ ID NO: 33, a *cry1Ab* gene coding region with a nucleic acid sequence as shown in SEQ ID NO: 34, an I-HSP70 intron with a nucleic acid sequence as shown in SEQ ID NO: 35, a chloroplast localized peptide CTP2 with a nucleic acid sequence as shown in SEQ ID NO: 36, a T-Tr7 terminator with a nucleic acid sequence as shown in SEQ ID NO: 37 that terminates the expression of the gene;
c) the third expression cassette comprises in operable linkage a P-CaMV35S promoter with a nucleic acid sequence as shown in SEQ ID NO: 38, a *pat* gene coding region with a nucleic acid sequence as shown in SEQ ID NO: 39, a T-35S polyA termination sequence with a nucleic acid sequence as shown in SEQ ID NO: 40 that terminates the expression of the gene.

The present invention further provides a DNA molecule having a sufficient length of contiguous nucleotides of SEQ ID NO: 10 to function as a DNA probe specific for SEQ ID NO: 10 in a sample of DNA derived from a corn plant, corn seed or corn cell, wherein the DNA probe comprises SEQ ID NO: 1 or SEQ ID NO: 2.

In one specific embodiment, the probe is SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 11 or SEQ ID NO: 12.

The present invention also provides a method of detecting the presence of DNA of transgenic corn event QY2569-42 in a sample, which comprises:
contacting a sample to be tested with at least two primers in a nucleic acid amplification reaction;
performing a nucleic acid amplification reaction;
detecting the presence of an amplification product;
the amplification product comprises (a) SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8 and/or SEQ ID NO: 10; or (b) a nucleic acid sequence complementary to (a);
the nucleic acid sequence is derived from transgenic corn event QY2569-42.

In one specific embodiment, the amplification product also comprises SEQ ID NO: 11 or a complementary sequence thereof and/or SEQ ID NO: 12 or a complementary sequence thereof.

In another specific embodiment, the two primers comprise SEQ ID NO: 13 and SEQ ID NO: 14, or else SEQ ID NO: 15 and SEQ ID NO: 16.

The present invention also provides a method of detecting the presence of DNA of transgenic corn event QY2569-42 in a sample, which comprises:
contacting a sample to be tested with a probe, wherein the probe comprises SEQ ID NO: 1, SEQ ID NO: 2, SEQ ID NO: 3, SEQ ID NO: 4, SEQ ID NO: 5, SEQ ID NO: 6, SEQ ID NO: 7 or SEQ ID NO: 8 or a complementary sequence thereof;
hybridizing the sample to be tested with the probe under stringent hybridization conditions;
detecting the hybridization between the sample to be tested and the probe.

In one specific embodiment, the probe also comprises SEQ ID NO: 11 or a complementary sequence thereof, or else SEQ ID NO: 12 or a complementary sequence thereof.

In another specific embodiment, at least one of the probes is labeled with at least one fluorophore.

The present invention also provides a method of detecting the presence of DNA of transgenic corn event QY2569-42 in a sample, which comprises:
contacting a sample to be tested with a marker nucleic acid molecule, wherein the marker nucleic acid molecule comprises SEQ ID NO: 1, SEQ ID NO: 2, SEQ ID NO: 3, SEQ ID NO: 4, SEQ ID NO: 5, SEQ ID NO: 6, SEQ ID NO: 7 or SEQ ID NO: 8 or a complementary sequence thereof;
hybridizing the sample to be tested with the marker nucleic acid molecule under stringent hybridization conditions;
detecting the hybridization between the sample to be tested and the marker nucleic acid molecule, and further determining the insect resistance and/or herbicide tolerance are genetically linked with the marker nucleic acid molecule by a marker assisted breeding analysis.

In one specific embodiment, the marker nucleic acid molecule also comprises SEQ ID NO: 11 or a complementary sequence thereof, or else SEQ ID NO: 12 or a complementary sequence thereof.

The present invention further provides a DNA detection kit, which comprises at least one DNA molecule. The DNA molecule comprises SEQ ID NO: 1, SEQ ID NO: 2, SEQ ID NO: 3, SEQ ID NO: 4, SEQ ID NO: 5, SEQ ID NO: 6, SEQ ID NO: 7 or SEQ ID NO: 8 or a complementary sequence thereof, which may function as one of DNA primers or a probe specific for transgenic corn event QY2569-42 or progeny thereof.

In one specific embodiment, when functioning as a probe, the DNA molecule also comprises SEQ ID NO: 11 or a complementary sequence thereof, or else SEQ ID NO: 12 or a complementary sequence thereof.

The present invention also provides a method for protecting a corn plant from insect infestation, comprising providing at least one transgenic corn plant cell in the diet of a target insect, wherein the transgenic corn plant cell sequentially comprises SEQ ID NO: 1, the nucleic acid sequence of position 777-11945 of SEQ ID NO: 10 and SEQ ID NO: 2, or, SEQ ID NO: 3, the nucleic acid sequence of position 777-11945 of SEQ ID NO: 10 and SEQ ID NO: 4, or, SEQ ID NO: 5, the nucleic acid sequence of position 777-11945 of SEQ ID NO: 10 and SEQ ID NO: 6 in its genome, or else the genome of the transgenic corn plant cell comprises SEQ ID NO: 10; the target insect that feeds on the transgenic corn plant cell is inhibited from further ingesting the corn plant.

The present invention also provides a method for protecting a corn plant from damage caused by herbicides, comprising applying a herbicide containing an effective amount of glufosinate to a field growing at least one transgenic corn plant, wherein the transgenic corn plant sequentially comprises SEQ ID NO: 1, the nucleic acid sequence of position 777-11945 of SEQ ID NO: 10 and SEQ ID NO: 2, or, SEQ ID NO: 3, the nucleic acid sequence of position 777-11945 of SEQ ID NO: 10 and SEQ ID NO: 4, or, SEQ ID NO: 5, the nucleic acid sequence of position 777-11945 of SEQ ID NO: 10 and SEQ ID NO: 6 in its genome, or else the genome of the transgenic corn plant comprises SEQ ID NO: 10; the transgenic corn plant has tolerance to glufosinate herbicide.

The present invention also provides a method of controlling weeds in a field growing corn plants, comprising applying a herbicide containing an effective amount of glufosinate to a field growing at least one transgenic corn plant, wherein the transgenic corn plant sequentially comprises SEQ ID NO: 1, the nucleic acid sequence of position 777-11945 of SEQ ID NO: 10 and SEQ ID NO: 2, or, SEQ ID NO: 3, the nucleic acid sequence of position 777-11945 of SEQ ID NO: 10 and SEQ ID NO: 4, or, SEQ ID NO: 5, the nucleic acid sequence of position 777-11945 of SEQ ID NO: 10 and SEQ ID NO: 6 in its genome, or else the genome of the transgenic corn plant comprises SEQ ID NO: 10; the transgenic corn plant has tolerance to glufosinate herbicide.

The present invention also provides a method of cultivating an insect-resistant corn plant, comprising planting at least one corn seed, wherein the genome of the corn seed comprises the nucleic acid sequence of a specific region and the nucleic acid sequence of the specific region sequentially comprises SEQ ID NO: 1, the nucleic acid sequence of position 777-11945 of SEQ ID NO: 10 and SEQ ID NO: 2, or, SEQ ID NO: 3, the nucleic acid sequence of position 777-11945 of SEQ ID NO: 10 and SEQ ID NO: 4, or, SEQ ID NO: 5, the nucleic acid sequence of position 777-11945 of SEQ ID NO: 10 and SEQ ID NO: 6, or else the nucleic acid sequence of the specific region comprises SEQ ID NO: 10; allowing the corn seed to grow into a corn plant;
infesting the corn plant with a target insect to harvest a plant having reduced plant damage compared with other plants that do not possess the nucleic acid sequence of the specific region.

The present invention also provides a method of cultivating a glufosinate-tolerant corn plant, comprising planting at least one corn seed, wherein the genome of the corn seed comprises the nucleic acid sequence of a specific region and the nucleic acid sequence of the specific region sequentially comprises SEQ ID NO: 1, the nucleic acid sequence of position 777-11945 of SEQ ID NO: 10 and SEQ ID NO: 2, or, SEQ ID NO: 3, the nucleic acid sequence of position 777-11945 of SEQ ID NO: 10 and SEQ ID NO: 4, or, SEQ ID NO: 5, the nucleic acid sequence of position 777-11945 of SEQ ID NO: 10 and SEQ ID NO: 6, or else the nucleic acid sequence of the specific region comprises SEQ ID NO: 10;
allowing the corn seed to grow into a corn plant;
spraying the corn plant with an effective amount of glufosinate to harvest a plant having reduced plant damage compared with other plants that do not possess the nucleic acid sequence of the specific region.

The present invention also provides a method of cultivating an insect-resistant and glufosinate-tolerant corn plant, comprising planting at least one corn seed, wherein the genome of the corn seed comprises the nucleic acid sequence of a specific region and the nucleic acid sequence of the specific region sequentially comprises SEQ ID NO: 1, the nucleic acid sequence of position 777-11945 of SEQ ID NO: 10 and SEQ ID NO: 2, or, SEQ ID NO: 3, the nucleic acid sequence of position 777-11945 of SEQ ID NO: 10 and SEQ ID NO: 4, or, SEQ ID NO: 5, the nucleic acid sequence of position 777-11945 of SEQ ID NO: 10 and SEQ ID NO: 6, or else the nucleic acid sequence of the specific region comprises SEQ ID NO: 10;
allowing the corn seed to grow into a corn plant;
spraying the corn plant with an effective amount of glufosinate to harvest a plant having reduced plant damage compared with other plants that do not possess the nucleic acid sequence of the specific region, wherein the plant having reduced plant damage is also resistant to feeding damage by insects.

The present invention also provides a method of producing an insect-resistant corn plant, comprising hybridizing a corn plant, whose genome sequentially comprises SEQ ID NO: 1, the nucleic acid sequence of position 777-11945 of SEQ ID NO: 10 and SEQ ID NO: 2, or, SEQ ID NO: 3, the nucleic acid sequence of position 777-11945 of SEQ ID NO: 10 and SEQ ID NO: 4, or, SEQ ID NO: 5, the nucleic acid sequence of position 777-11945 of SEQ ID NO: 10 and SEQ ID NO: 6, with another corn plant, thereby producing a large number of progeny plants; selecting a progeny plant whose genome comprises the nucleic acid sequence of a specific region, wherein the nucleic acid sequence of the specific region sequentially comprises SEQ ID NO: 1, the nucleic acid sequence of position 777-11945 of SEQ ID NO: 10 and SEQ ID NO: 2, or, SEQ ID NO: 3, the nucleic acid sequence of position 777-11945 of SEQ ID NO: 10 and SEQ ID NO: 4, or, SEQ ID NO: 5, the nucleic acid sequence of position 777-11945 of SEQ ID NO: 10 and SEQ ID NO: 6, or else the nucleic acid sequence of the specific region comprises SEQ ID NO: 10 and the progeny plant has reduced plant damage caused by insect feeding.

In one specific embodiment, the method comprises sexually crossing a first parental corn plant of transgenic corn event QY2569-42 that is insect-resistant with a second parental corn plant that lacks insect resistance, thereby producing a large number of progeny plants;
infesting the progeny plants with a target insect;
selecting the progeny plant having reduced plant damage compared with other plants that do not possess the nucleic acid sequence of the specific region.

The present invention also provides a method of producing a glufosinate-tolerant corn plant, comprising hybridizing a corn plant, whose genome sequentially comprises SEQ ID NO: 1, the nucleic acid sequence of position 777-11945 of SEQ ID NO: 10 and SEQ ID NO: 2, or, SEQ ID NO: 3, the nucleic acid sequence of position 777-11945 of SEQ ID NO: 10 and SEQ ID NO: 4, or, SEQ ID NO: 5, the nucleic acid sequence of position 777-11945 of SEQ ID NO: 10 and SEQ ID NO: 6, with another corn plant, thereby producing a large number of progeny plants; selecting a progeny plant whose genome comprises the nucleic acid sequence of a specific region, wherein the nucleic acid sequence of the specific region sequentially comprises SEQ ID NO: 1, the nucleic acid sequence of position 777-11945 of SEQ ID NO: 10 and SEQ ID NO: 2, or, SEQ ID NO: 3, the nucleic acid sequence of position 777-11945 of SEQ ID NO: 10 and SEQ ID NO: 4, or, SEQ ID NO: 5, the nucleic acid sequence of position 777-11945 of SEQ ID NO: 10 and SEQ ID NO: 6, or else the nucleic acid sequence of the specific region comprises SEQ ID NO: 10 and the progeny plant has glufosinate tolerance.

In one specific embodiment, the method comprises sexually crossing a first parental corn plant of transgenic corn event QY2569-42 that is glufosinate-tolerant with a second parental corn plant that lacks glufosinate tolerance, thereby producing a large number of progeny plants;
treating the progeny plants with glufosinate herbicide;
selecting the progeny plant that is glufosinate-tolerant.

The present invention also provides a method of producing a corn plant that is resistant to insects and tolerant to glufosinate herbicide application, comprising sexually crossing a first parental corn plant of transgenic corn event QY2569-42 that is glufosinate-tolerant and insect-resistant with a second parental corn plant that lacks glufosinate tolerance and/or insect resistance, thereby producing a large number of progeny plants;
treating the progeny plants with glufosinate;
selecting the progeny plant that is glufosinate-tolerant, wherein the progeny plant that is glufosinate-tolerant is also resistant to feeding damage by insects.

The present invention also provides a method for improving the tolerance of a corn plant, comprising:
a) constructing the aforementioned DNA construct;
b) inserting the DNA construct into the genome of a corn cell;
c) regenerating the corn cell into a corn plant; and
d) selecting a corn plant comprising the DNA construct.

In one specific embodiment, improvement of tolerance of a corn plant includes resistance to an effective amount of at least one herbicide and the herbicide is preferably glufosinate; and/or
improvement of tolerance of a corn plant includes resistance to at least one insect and the insect is preferably *Ostrinia furnacalis, Mythimna separata, Spodoptera frugiperda* and/or *Helicoverpa armigera.*

The present invention also provides a composition produced from transgenic corn event QY2569-42, wherein the composition is corn flour, corn meal, corn oil, corn silk or corn starch.

The present invention also provides an agricultural product or commodity produced from transgenic corn event QY2569-42, wherein the agricultural product or commodity is corn flour, corn meal, corn oil, corn starch, corn gluten, corn cake, cosmetics or filling agents.

The present invention also provides a plant cell, plant part, plant or seed, comprising the aforementioned nucleic acid sequence.

The present invention also provides a non-living plant material comprising the aforementioned nucleic acid sequence.

Some terms used in this specification are defined as follows.

The "corn" of the present invention refers to *Zea mays* and includes all plant varieties that can be bred with corn plants including wild corn species.

The term "comprising" of the present invention refers to "including but not limited to".

The term "insecticidal" or "insect-resistant" of the present invention refers to being toxic to agricultural crop pests, so as to "control" and/or "prevent" agricultural crop pests. Preferably, the term "insecticidal" or "insect-resistant" refers to killing agricultural crop pests. The above-mentioned pests include lepidopterans.

The "lepidopteran", the scientific name of which is Lepidoptera, includes two categories of insects, moths and butterflies, and is an order with the most agricultural and forestry pests such as *Ostrinia nubilalis, Spodoptera frugiperda, Helicoverpa armigera, Mythimna separata, Athetis lepigone, Dichocrocis punctiferalis,* etc.

The "herbicide" of the present invention refers to an active ingredient capable of killing or controlling or being detrimental to plant growth. The term "herbicide-tolerant", "herbicide-resistant", "herbicide tolerance" or "herbicide resistance" in the present invention refers to that the plant continues to grow even after being treated with a herbicide which can kill an ordinary or wild-type plant or inhibit plant growth, or, make the growth ability of a plant weaker compared with a wild-type plant or stop plant growth. The above-mentioned herbicide includes glutamine synthase inhibitor herbicides, e.g., glufosinate.

The term "gene" refers to a nucleic acid fragment expressing a specific protein, including regulatory sequences before (5' non-coding sequence) and after (3' non-coding sequence) a coding sequence. A "native gene" refers to a gene that is naturally found to have its own regulatory sequences. A "chimeric gene" refers to any gene that is not a native gene, comprising regulatory and coding sequences that are not naturally found. An "endogenous gene" refers to a native gene in its natural location in the genome of an organism. An "exogenous gene" refers to a foreign gene that exists currently and did not exist previously in the genome of an organism, and also refers to a gene introduced into a recipient cell through transgenic procedures. An exogenous gene may comprise a native gene or chimeric gene inserted into a non-natural organism. A "transgene" refers to a gene that has been introduced into a genome by transformation procedures. A site where a recombinant DNA has been inserted in a plant genome can be referred to as "insertion site" or "target site".

The term "nucleotide sequence" or "nucleic acid sequence" can be used interchangeably, referring to an oligonucleotide, nucleotide or polynucleotide and a fragment or part thereof, which may be single-stranded or double-stranded, representing a sense or antisense strand. A nucleic acid includes DNA, RNA or a hybrid of both, and can be of a natural or synthetic origin. For example, a nucleic acid may include a mRNA or cDNA. A nucleic acid may include a nucleic acid that has been amplified (e.g., by a polymerase chain reaction). The one-letter symbols for nucleotides are as set forth in Appendix A, Chapter 2422 of the Manual of Patent Examining Procedure by the U.S. Patent and Trademark Office (USPTO).

The term "transgenic" plant refers to a plant comprising a heterologous polynucleotide. Preferably, a heterologous polynucleotide is stably integrated into a genome, allowing the polynucleotide to be passed on to successive generations. A heterologous polynucleotide can be integrated into a genome alone or as a part of a recombinant expression cassette. The term "transgenic" is used herein to refer to any cell, cell line, callus, tissue, plant part or plant whose genotype is altered due to the presence of a heterologous nucleic acid, including those originally altered transgenic organisms or cells, as well as those resulting from crossing or asexual propagation of originally altered transgenic organisms or cells. The term "transgenic" as used herein is not intended to include altering a genome (chromosomal or extrachromosomal) by conventional plant breeding methods (e.g., crossing) or by naturally occurring events (e.g., self-fertilization, random cross-fertilization, non-recombinant viral infection, non-recombinant bacterial transformation, non-recombinant transposition, or spontaneous mutation).

A transgenic "event" is produced by transformation of plant cells with heterologous DNA, i.e., a nucleic acid construct that includes a transgene of interest, regeneration of a population of plants resulting from the insertion of the transgene into the genome of the plant, and selection of a particular plant characterized by insertion into a particular genome location. The term "event" refers to the original transformant and progeny of the transformant that contain the heterologous DNA. The term "event" also refers to progeny produced by a sexual outcross between the transformant and another variety that includes the genomic/transgene DNA. Even after repeated back-crossing to a recurrent parent, the inserted transgene DNA and flanking genomic DNA (genomic/transgene DNA) from the transformed parent is present in the progeny of the cross at the same chromosomal location. The term "event" also refers to DNA from the original transformant and progeny thereof comprising the inserted DNA and flanking genomic sequence immediately adjacent to the inserted DNA that would be expected to be transferred to a progeny that receives inserted DNA including the transgene of interest as the result of a sexual cross of one parental line that includes the inserted DNA (e.g., the original transformant and progeny resulting from selfing) and a parental line that does not contain the inserted DNA.

The term "inserted DNA" or "insertion sequence" refers to heterologous DNA in an expression cassette used for transformation of plant materials. Inserted DNA is derived from T-DNA, which is contained in a binary vector used in *Agrobacterium*-mediated transformation of a plant.

The "flanking DNA" or "flanking sequence" of the present invention may comprise either a genome naturally present in an organism such as a plant, or exogenous (heterologous) DNA introduced via the transformation process, e.g., a fragment associated with the transformation event. Thus, flanking DNA may comprise a combination of natural and exogenous DNA. In the present invention, a "flanking region" or "flanking sequence" or "genomic border region" or "genomic border sequence" refers to a sequence of at least 3, 5, 10, 11, 15, 20, 50, 100, 200, 300, 400, 1000, 1500, 2000, 2500 or 5000 base pairs or greater, which is located either immediately upstream or downstream of, and contiguous with, the original foreign inserted DNA molecule. When this flanking region is located downstream it may also be referred to as "left border flank" or "3' flank" or "3' genomic border region" or "genomic 3' border sequence", and the like. When this flanking region is located upstream it may also be referred to as the "right border flank" or "5' flank" or "5' genomic border region" or "genomic 5' border sequence", and the like.

The term "junction" refers to a point where two specific DNA fragments join. For example, a junction exists where inserted DNA joins flanking DNA. A junction point also exists in a transformed organism where two DNA fragments join together in a manner that is modified from that found in the native organism. "Junction DNA" or "junction sequence" refers to DNA that comprises a junction point.

A "junction sequence" spans the point at which DNA inserted into the genome is linked to DNA from the corn native genome flanking the insertion point, the identification or detection of one or the other junction sequences in a plant's genetic material being sufficient to be diagnostic for the event. Included are the DNA sequences that span the insertions in herein-described corn events and similar lengths of flanking DNA. Specific examples of such diagnostic sequences are provided herein; however, other sequences that overlap the junctions of the insertions, or the junctions of the insertions and the genomic sequence, are also diagnostic and could be used according to the present invention.

The term "probe" is an isolated nucleic acid molecule which binds to a conventional detectable marker or reporter molecule thereon, for example, a radioisotope, a ligand, a chemiluminescent agent or enzymes. Such probe is complementary to a strand of the target nucleic acid. In the present invention, the probe is complementary to a DNA strand from the genome of transgenic corn event QY2569-42, irrespective of the genomic DNA being derived from transgenic corn event QY2569-42 or seed thereof or derived from the plants or seeds or extracts of transgene corn event QY2569-42. The probe of the present invention includes not only deoxyribonucleic acid or ribonucleic acid but also polyamides and other probe materials which specifically bind to the target DNA sequence and can be used to detect the presence of the target DNA sequence.

The term "primer" is an isolated nucleic acid molecule that binds to a complementary target DNA strand by nucleic acid hybridization and annealing, forms a hybrid between the primer and the target DNA strand, then extends along the target DNA strand under the action of a polymerase (e.g., DNA polymerase). The primer pairs of the present invention relate to their application in the amplification of target nucleic acid sequences, for example, by polymerase chain reaction (PCR) or other conventional nucleic acid amplification methods.

The nucleic acid probes and primers of the present invention hybridize under stringent conditions to a target DNA molecule. Any conventional nucleic acid hybridization or amplification method can be used to identify the presence of DNA from a transgenic plant in a sample. Polynucleic acid molecules, which are also referred to as nucleic acid segments, or fragments thereof are capable of specifically hybridizing to other nucleic acid molecules under certain circumstances.

As used herein, if two polynucleotide molecules are capable of forming an anti-parallel double-stranded nucleic acid structure, the two molecules are capable of specifically hybridizing to one another. If two nucleic acid molecules exhibit complete complementarity, one of the nucleic acid molecules is said to be "complementary" to the other nucleic acid molecule. As used herein, molecules are said to exhibit "complete complementarity" when every nucleotide in one of the molecules is complementary to nucleotides in the other molecule. Two molecules are said to be "minimally complementary" if they can hybridize to one another with sufficient stability to permit them to remain bound to one another under at least conventional "low-stringency" conditions. Similarly, the molecules are said to be "complementary" if they can hybridize to one another with sufficient stability to permit them to remain bound to one another under conventional "high-stringency" conditions. Conventional stringency conditions are described by Sambrook *et al.,* 1989, and by Haymes et al., In: Nucleic Acid Hybridization, A Practical Approach, IRL Press, Washington, DC (1985). Departures from complete complementarity are therefore permissible, as long as such departures do not completely preclude the capacity of the molecules to form a double-stranded structure. In order for a nucleic acid molecule to serve as a primer or probe, it need only be sufficiently complementary in sequence to be able to form a stable double-stranded structure under the particular solvent and salt concentrations employed.

The term "amplicon" refers to the nucleic acid amplification product of a target nucleic acid sequence that is part of a nucleic acid template. The "amplicon" and "amplification product" of the present invention may be used interchangeably. For example, in order to determine whether a corn plant is resulted from a sexual cross by the transgenic corn event QY2569-42 of the present invention, or whether a corn sample collected from a field contains the transgenic corn event QY2569-42, or whether a corn extract, e.g., corn meal, powder or oils, contains the transgenic corn event QY2569-42, the DNA extracted from a corn plant tissue sample or extract may produce amplicons diagnostic for the presence of DNA of transgenic corn event QY2569-42 by a nucleic acid amplification method using a primer pair. The primer pair comprises a first primer derived from a flanking sequence adjacent to the insertion site of the inserted exogenous DNA in the plant genome and a second primer derived from the inserted exogenous DNA. The amplicon has a certain length and a sequence that is also diagnostic for the transgenic corn event QY2569-42. The length range of the amplicon may be the binding length of the primer pair plus one nucleotide base pair, preferably plus about 50 nucleotide base pairs, more preferably plus about 250 nucleotide base pairs, most preferably plus about 450 nucleotide base pairs or more.

The term "plant" is used in its broadest sense as it pertains to organic matter and is intended to encompass eukaryotic organisms that belong to the Kingdom Plantae, examples of which include but are not limited to vascular plants, vegetables, grains, flowers, trees, herbs, bushes, grasses, vines, ferns, mosses, fungi and algae, etc., as well as clones, offsets, and parts of plants used for asexual propagation (e.g., cuttings, pipings, shoots, rhizomes, underground stems, clumps, crowns, bulbs, corms, tubers, rhizomes, plants/tissues produced in tissue culture, etc.). The term "plant" further encompasses whole plants, ancestors and progeny of plants and plant parts, including seeds, shoots, stems, leaves, roots (including tubers), flowers, florets, fruits, pedicles, peduncles, stamen, anther, stigma, style, ovary, petal, sepal, carpel, root tip, root cap, root hair, leaf hair, seed hair, pollen grain, microspore, cotyledon, hypocotyl, epicotyl, xylem, phloem, parenchyma, endosperm, companion cells, guard cells, and any other known organs, tissues, and cells of a plant, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprise the gene/nucleic acid of interest.

The term "plant tissue" or "plant part" includes a plant cell, protoplast, plant tissue culture, plant callus, plant piece as well as a plant embryo, pollen, ovule, seed, leaf, stem, flower, branch, shoot, fruit, pit, ears, root, root tip, anther, etc.

The term "plant cell" should be understood as any cell derived from or found in a plant, which is capable of forming, for example, undifferentiated tissues such as calli, differentiated tissues such as embryos, constituent parts of a plant, plants, or seeds.

The genomes of the plants, plant tissues or plant cells in the present invention refer to any genetic material in the plants, plant tissues or plant cells, and include the genomes of nuclei, plastids and mitochondria.

The term "expression cassette" refers to the complete elements required for expressing a gene, which contains a nucleotide sequence that can encode the protein of interest, and the nucleotide sequence comprises a start codon and a stop codon.

The term "kit" refers to any manufacture (e.g., a package or a container) including at least one device. A kit can further include the method described herein, or instructions for use, supplemental reagents and/or components or modules used in steps thereof.

In some embodiments, the kit also comprises one or more of nucleic acid extraction reagents, reagents for nucleic acid amplification, positive controls and negative controls.

The term "DNA construct" or "recombinant vector" means a vector comprising a heterologous or recombinant nucleotide sequence. The term "vector" refers to a nucleic acid fragment or polynucleic acid fragment used for introducing or transferring one or more nucleic acids or one or more polynucleic acids into target cells or tissues.

Methods of vector transformation include introducing recombinant plasmids into plants using methods such as *Agrobacterium*-mediated transformation, electroporation, microparticle bombardment, polyethylene glycol (PEG)-medium absorption, etc.

The recipients of plant transformation in the present invention include plant cells (including suspension culture cells), protoplasts, calli, hypocotyls, seeds, cotyledons, shoots, and mature plant bodies.

The scope of transgenic plants includes not only the plant body obtained at the time of gene introduction, but also clones and progeny thereof (T1 generation, T2 generation or subsequent generations). The scope of the present invention also includes all mutants and variants, by crossing and fusion of the above-mentioned transgenic plants, exhibiting the characteristics of the first-generation transgenic plants. The scope of the present invention also includes a part of a plant, such as seed, flower, stem, fruit, leaf, root, tuber, tuberous stem, which are derived from plants that have been genetically modified in advance by the methods mentioned in the present invention or from progeny thereof, at least consisting of a portion of transgenic cells.

The present invention relates to the identification of such flanks, junctions and insertion sequences. The present invention also includes the PCR primers and amplicons designed based on the above sequences. The PCR analysis method of the present invention using amplicons that span across inserted DNA and its borders can be used to detect or identify commercialized transgenic corn varieties or lines derived from the proprietary transgenic corn of the present invention.

### Brief Description of Sequence

SEQ ID NO: 1: 10 nucleotides at each side of the insertion site of the 5' transgenic fragment and the corn genomic DNA in the transgenic corn event QY2569-42;
SEQ ID NO: 2: 10 nucleotides at each side of the insertion site of the 3' transgenic fragment and the corn genomic DNA in the transgenic corn event QY2569-42;
SEQ ID NO: 3: 20 nucleotides at each side of the insertion site of the 5' transgenic fragment and the corn genomic DNA in the transgenic corn event QY2569-42;
SEQ ID NO: 4: 20 nucleotides at each side of the insertion site of the 3' transgenic fragment and the corn genomic DNA in the transgenic corn event QY2569-42;
SEQ ID NO: 5: 50 nucleotides at each side of the insertion site of the 5' transgenic fragment and the corn genomic DNA in the transgenic corn event QY2569-42;
SEQ ID NO: 6: 50 nucleotides at each side of the insertion site of the 3' transgenic fragment and the corn genomic DNA in the transgenic corn event QY2569-42;
SEQ ID NO: 7: A sequence with a length of 1269 nucleotides at the 5' terminus of the insertion sequence near the insertion junction site in the transgenic corn event QY2569-42;
SEQ ID NO: 8: A sequence with a length of 3057 nucleotides at the 3' terminus of the insertion sequence near the insertion junction site in the transgenic corn event QY2569-42;
SEQ ID NO: 9: A transgenic corn event QY2569-42 insertion sequence derived from the T-DNA region of the pQY002569 vector;
SEQ ID NO: 10: Entire T-DNA sequence, 5' and 3' flanking corn genome sequences;
SEQ ID NO: 11: A sequence which is located within SEQ ID NO: 7 and is the amplicon of primers SEQ ID NO: 13 and SEQ ID NO: 14;
SEQ ID NO: 12: A sequence which is located within SEQ ID NO: 8 and is the amplicon of primers SEQ ID NO: 15 and SEQ ID NO: 16;
SEQ ID NO: 13: A primer on the 5' flanking genome sequence;
SEQ ID NO: 14: A primer on the T-DNA and paired with SEQ ID NO: 13;
SEQ ID NO: 15: A primer on the 3' flanking genome sequence;
SEQ ID NO: 16: A primer on the T-DNA and paired with SEQ ID NO: 15;
SEQ ID NO: 17: PCR primer 1 for detecting *mvip3Aa20*;
SEQ ID NO: 18: PCR primer 2 for detecting *mvip3Aa20*;
SEQ ID NO: 19 PCR primer 1 for detecting *Cry1Ab;*
SEQ ID NO: 20 PCR primer 2 for detecting *Cry1Ab;*
SEQ ID NO: 21: PCR primer 1 for detecting *pat;*
SEQ ID NO: 22: PCR primer 2 for detecting *pat;*
SEQ ID NO: 23: Probe primer 1 for *mvip3Aa20* in Southern blotting;
SEQ ID NO: 24: Probe primer 2 for *mvip3Aa20* in Southern blotting;
SEQ ID NO: 25 Probe primer 1 for *Cry1Ab* in Southern blotting;
SEQ ID NO: 26 Probe primer 2 for *Cry1Ab* in Southern blotting;
SEQ ID NO: 27: Probe primer 1 for *pat* in Southern blotting;
SEQ ID NO: 28: Probe primer 2 for *pat* in Southern blotting;
SEQ ID NO: 29: The nucleic acid sequence of the promoter OsUbi2;
SEQ ID NO: 30: The nucleic acid sequence of the *mVip3Aa20* gene coding region;
SEQ ID NO: 31: The nucleic acid sequence of the chloroplast leading peptide CTP-TS-SSU;
SEQ ID NO: 32: The nucleic acid sequence of the terminator T-Ara5;
SEQ ID NO: 33: The nucleic acid sequence of the promoter P-FMV;
SEQ ID NO: 34: The nucleic acid sequence of the *cry1Ab* gene coding region;
SEQ ID NO: 35: The nucleic acid sequence of the intron I-HSP70;
SEQ ID NO: 36: The nucleic acid sequence of the chloroplast-localized peptide CTP2;
SEQ ID NO: 37: The nucleic acid sequence of the terminator T-Tr7;
SEQ ID NO: 38: The nucleic acid sequence of the promoter P-CaMV35S;
SEQ ID NO: 39: The nucleic acid sequence of the *pat* gene coding region;
SEQ ID NO: 40: The nucleic acid sequence of the termination sequence T-35S polyA.

### Description of Figures

Figure 1 shows the schematic of the pQY002569 vector.
Figure 2 shows the structural schematic of the conjugation sites between the transgene insertion sequences and the corn genome of a nucleic acid sequence for detecting the corn plant QY2569-42 and a detection method thereof.
Figure 3 shows the amplification results of the genes of interest in T2-T4 generations of QY2569-42. M: Marker; 1: Single strain of T2 generation of QY2569-42; 2: Single strain of T3 generation of QY2569-42; 3: Single strain of T4 generation of QY2569-42; P: Plasmid pQY002569; N: Non-transgenic corn ZM11.
Figure 4 shows the analysis of the enzyme restriction sites of insertion sequences.
Figure 5 shows the specific PCR amplification results of T2-T4 generations of QY2569-42. M: Marker; 1: Single strain of T2 generation of QY2569-42; 2: Single strain of T3 generation of QY2569-42; 3: Single strain of T4 generation of QY2569-42; P: Plasmid pQY002569; N: Non-transgenic corn ZM11.
Figure 6 shows the chromosomal locating schematic of the QY2569-42 insertion sequence.
Figure 7 shows the results of transgenic corn event QY2569-42 inoculated with *Spodoptera frugiperda* at whorl stage and ear stage in field; CK is ZM11.
Figure 8 shows the results of transgenic corn event QY2569-42 inoculated with *Ostrinia furnacalis* at whorl stage and ear stage in field; CK is ZM11.
Figure 9 shows the results of transgenic corn event QY2569-42 inoculated with *Mythimna separata* at whorl stage in field; CK is ZM11.
Figure 10 shows the results of transgenic corn event QY2569-42 inoculated with *Helicoverpa armigera* at ear stage in field; CK is ZM11.

### Detailed Embodiments of the Invention

The following examples are put forth so as to provide those skilled in the art with a complete disclosure and description of how to prepare and use the present invention, and are not intended to limit the scope of what the inventors regard as their invention, nor are they intended to represent or imply that the experiments below are all of or the only experiments performed. It will be appreciated by those skilled in the art that numerous variations and/or modifications may be made to the invention as shown in the specific aspects without departing from the spirit or scope of the invention as broadly described. Therefore, the aspects herein are to be considered in every respect as illustrative rather than restrictive.

### Example 1 Construction of transgenic vector pQY002569

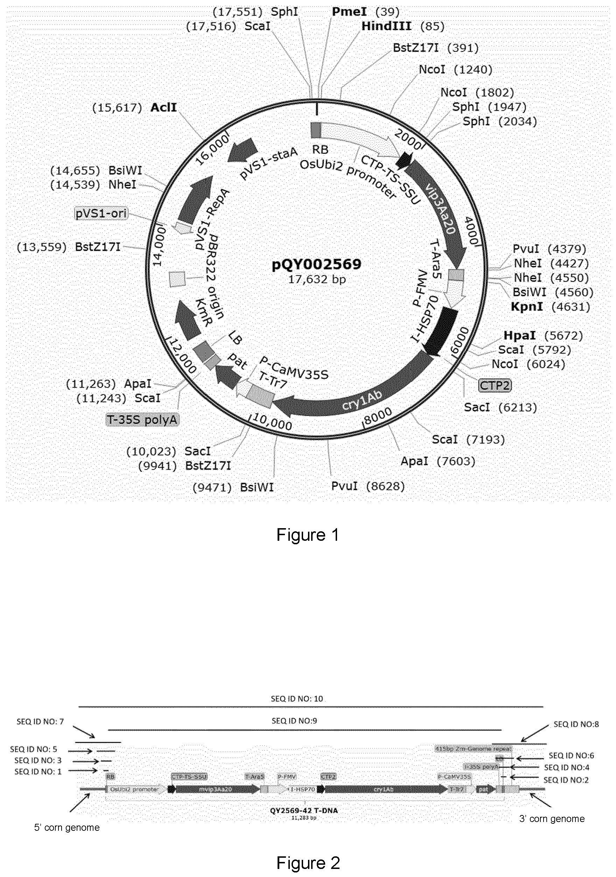

The pQY002569 plasmid was 17,632 bp in size and was artificially constructed based on pCAMBIA1300. The pCAMBIA1300 is a commonly used plant expression vector, without a *gus* reporter gene on the vector backbone, whose replication origin is derived from *E. coli,* and backbone is derived from pUC18. It contains T-DNA left border (LB) and right border (RB), kanamycin resistance gene (KmR), replication origin derived from pBR322 (pBR322 origin), replicon (pVS1 rep) and pVS1 stable region (pVS1 staA) derived from pVS1. The hygromycin resistance gene on the pCAMBIA1300 plasmid was removed by enzyme digestion, and then 3 artificially synthesized expression cassettes of the genes of interest (OsUbi2 *promoter*::CTP-TS-SSU::*mvip3Aa20*::T-Ara5; P-FMV::I-HSP70::CTP2::*cry1Ab*::T-Tr7; P-CaMV35S::*pat*::T-35S polyA) were introduced by the Golden Gate method to form the final vector pQY002569 (as shown in Figure 1).

### Example 2 Transformation of pQY002569 vectors and selection of transgenic plants

The QY2569 insect-resistant and herbicide-tolerant corn was obtained by introducing the insect resistance genes *mvip3Aa20, cry1Ab* and the herbicide tolerance gene *pat* into a non-transgenic corn ZM11 through *Agrobacterium*-mediated transformation with immature embryos. Wherein, the corn inbred line ZM11,which retained the disease resistance characteristic of Q319, was obtained by the steps of crossing Hill with Q319, backcrossing the hybrid with Q319 for two generations, selecting offspring that were easy to genetically transform, and then performing self-crossing on the offspring.

By PCR and Southern blotting analysis, the presence of *mvip3Aa20, cry1Ab* and herbicide tolerance gene *pat* in the transgenic corn plant was detected and the copy numbers of the transgenes in the insect-resistant and glufosinate herbicide-tolerant strains were characterized. By screening, the corn event QY2569-42 was selected to be excellent in its performance with single-copy transgene as well as good insect resistance, glufosinate herbicide tolerance and agronomic traits.

### Example 3 Genetic elements of transgenic corn event QY2569-42 and specificity detection method

The transgenic corn event QY2569-42 insertion sequence was derived from the T-DNA region of the pQY002569 vector, with a size of 11283 bp (SEQ ID NO: 9). The insertion sequence comprised a T-DNA fragment missing part of RB and part of LB, whose structure is shown in Figure 2. Genetic elements contained in the insertion sequence are shown in Table 1.

**Table 1 Genetic elements contained in the QY2569-42 insertion sequence**

| **Element** | **Size (bp)** | **Position located on SEQ ID NO: 10** | **Function** | **Source** |
|---|---|---|---|---|
| Zm-gDNA 5' flanking | 720 | 1-720 | Corn genomic DNA | Corn |
| T-DNA right boundary sequence (RB) | 37 | 721-757 | T-DNA right boundary sequence | *Agrobacterium* |
| Intervening sequence | 19 | 758-776 | Sequence for DNA cloning | |
| OsUbi2 promoter | 1708 | 777-2484 | Rice Ubiquitin2 promoter, initiating the expression of the *mvip3Aa20* gene | Rice |
| CTP-TS-SSU | 237 | 2488-2724 | Chloroplast-localized peptide, leading *mVip3Aa20* mature protein into chloroplasts | Corn |
| *mvip3Aa20* | 2373 | 2728-5100 | Encoding the insecticidal protein *mVip3Aa20* | *Bacillus thuringiensis* |
| T-Ara5 | 213 | 5101-5313 | Rice *RA5B* gene terminator | Rice |
| P-FMV | 565 | 5320-5884 | Figwort mosaic virus 35S promoter, initiating the expression of the *cry1Ab* gene | Figwort mosaic virus |
| Intervening sequence | 21 | 5885-5905 | Sequence for DNA cloning | |
| I-HSP70 | 804 | 5906-6709 | Intron of corn *hsp70* gene, stabilizing the transcription of the *cry1Ab* gene | Corn |
| CTP2 | 228 | 6713-6940 | Chloroplast-localized peptide, leading Cry1Ab mature protein into chloroplasts | *Arabidopsis thaliana* |
| *cry1Ab* | 3471 | 6941-10411 | Encoding insect-resistant protein Cry1Ab | *Bacillus thuringiensis* |
| T-Tr7 | 505 | 10412-10916 | *Agrobacterium transcript7* gene terminator | *Agrobacterium* |
| P-CaMV35S | 293 | 10920-11212 | Cauliflower mosaic virus 35S promoter, initiating the expression of the *pat* gene | Cauliflower mosaic virus |
| *pat* | 555 | 11216-11770 | Encoding herbicide-resistant protein PAT | *Streptomyces viridochromogenes* |
| T-35S polyA | 175 | 11771-11945 | Cauliflower mosaic virus 35S transcription termination signal | Cauliflower mosaic virus |
| Intervening sequence | 38 | 11946-11983 | Sequence for DNA cloning | |
| DNA left boundary sequence | 20 | 11984-12003 | T-DNA left boundary sequence | *Agrobacterium* |
| (LB) | | | | |
| Randomly inserted base | 2 | 12004-12005 | Randomly insertion sequence | |
| Zm-gDNA repeat | 415 | 12006-12420 | Repeats of 415 bp of corn genomic DNA at Zm-gDNA 5' flanking | Corn |
| Zm-gDNA 3' flanking | 2757 | 12004-14760 | Corn genomic DNA | Corn |

According to the inserted fragment and the genomic sequences flanking the inserted fragment, related primers were designed, a detection method for inserted elements and a specificity detection method of transgenic corn event QY2569-42 were developed.

### 1. PCR detection of exogenous genes

DNA was extracted and purified in accordance with the National Agriculture Industry Standard NY/T674 of the People's Republic of China. Using the genomic DNA of the leaves of T2-T4 generations of the transgenic corn QY2569-42 as a template, the exogenous genes *mvip3Aa20, cry1Ab* and *pat* were amplified to determine whether the exogenous genes had been integrated into the corn genome. The primers for amplification are as shown in Table 2:

**Table 2 Data of PCR primers for the exogenous genes of the transgenic corn event QY2569-42**

| **Target sequence** | **Sequence No.** | **Primer sequence** | **Size of amplification products** |
|---|---|---|---|
| *mvip3Aa20* | SEQ ID NO: 17 | 5'-GCTGTTTGATCCGTTGTTGTGT-3' | 585 bp |
| | SEQ ID NO: 18 | 5'-GAGGATCTCCTTGCTCAGCT-3' | |
| *cry1Ab* | SEQ ID NO: 19 | 5'-GCCTTCTCCCTAGTGTTGAC-3' | 565 bp |
| | SEQ ID NO: 20 | 5'-CGCTGGTTGATGAGTTGCTC-3' | |
| *pat* | SEQ ID NO: 21 | 5'-CCCGATCCTACCTGTCACTT-3' | 794 bp |
| | SEQ ID NO: 22 | 5'-CTCTGCCAGAACCCGACGTC-3' | |

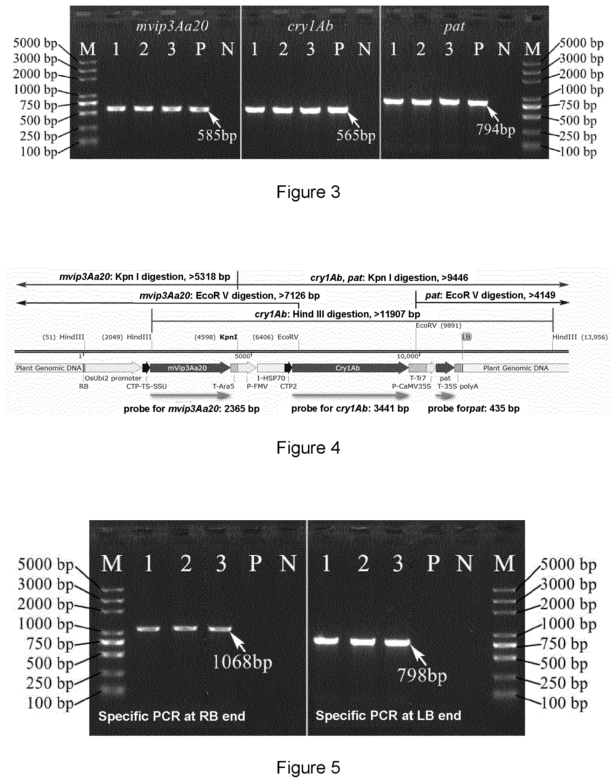

The amplification results are as shown in Figure 3: using the genomic DNA of T2-T4 generation plants of QY2569-42 and ZM11 (negative control) as templates, the expected fragments of the exogenous genes *mvip3Aa20, cry1Ab* and *pat* were all amplified from T2-T4 generations of QY2569-42, with sizes of 585 bp, 565 bp and 794 bp, respectively. No exogenous gene bands were amplified from the negative control; exogenous gene bands were amplified from the positive control (transformed vector).

### 2. Southern blotting of exogenous genes

Restriction site analysis was performed on the sequences of the inserted elements on the vector to determine the restriction sites used in Southern blotting. According to the restriction sites and probe positions used (Figure 4), the sizes of hybridization fragments were accurately predicted. The expected hybridization fragments and the actual hybridization fragments were completely identical in size.

Using the pQY002569 plasmids as templates and the specific primers of *mvip3Aa20, cry1Ab* and *pat* genes, respectively, digoxigenin-labeled specific probes were prepared using Roche DIG probe labeling kit according to the instructions, hybridized to the digested corn genomic DNA for determination. The primer sequences are as follows:

**Table 3 Data of PCR primers for Southern probes**

| **Probe** | **Sequence No.** | **Primer sequence** | **Size of probes** |
|---|---|---|---|
| *mvip3Aa20* | SEQ ID NO: 23 | 5'-GAACAAGAACAACACGAAGCTCT-3' | 2365 bp |
| | SEQ ID NO: 24 | 5'-CTTGATGGAGACGTCGTAGAAGT-3' | |
| *cry1Ab* | SEQ ID NO: 25 | 5'-CATTCCCTACAACTGCCTGTCT-3' | 3441 bp |
| | SEQ ID NO: 26 | 5'-CATTACTCTTCCATGAGCAGAAGCT-3' | |
| *pat* | SEQ ID NO: 27 | 5'-GTGCGACATCGTCAACCACT-3' | 435 bp |
| | SEQ ID NO: 28 | 5'-CTCTGCCAGAACCCGACGTC-3' | |

The results of Southern blotting demonstrated that, the probes for *mvip3Aa20, cry1Ab* and *pat* genes all obtained the hybridization results consistent with expectations, and only 1 copy was inserted into the corn genome without other unexpected fragments inserted.

### 3. Specificity detection of transgenic corn event QY2569-42

Genome walking was performed on the transgenic corn event QY2569-42 to obtain a 5' flanking sequence of 720 bp which was within SEQ ID NO: 7 and a 3' flanking sequence of 2757 bp which was within SEQ ID NO: 8. Amplicons were generated by using at least one primer derived from SEQ ID NO: 7 or SEQ ID NO: 8. The primer generated amplicons diagnostic for the transgenic corn event QY2569-42 by PCR (Table 4).

**Table 4. Data of specific PCR primers for flanking sequences of transgenic corn QY2569-42**

| **Target sequence** | **Sequence No.** | **Primer sequence** | **Size of amplification products** |
|---|---|---|---|
| 5' flanking | SEQ ID NO: 13 | 5'-CGACGCACGACATGCAAACA-3' | 1068 bp (SEQ ID NO: |
| PCR primer | SEQ ID NO: 14 | 5'-CCGTTTGGTACACGTCTCGT-3' | 11) |
| 3' flanking | SEQ ID NO: 16 | 5'-CTGGCAGAGGGACTTCGAGCT-3' | 798 bp (SEQ ID NO: 12) |
| PCR primer | SEQ ID NO: 15 | 5'-CCCTTCCCCTCCTCTCTCTT-3' | |

Specifically, a PCR product was generated from the 5' terminus of the transgene insertion sequence; a primer (SEQ ID NO: 13) hybridized to the genomic DNA sequence flanking the 5' terminus of the transgene insertion sequence and a paired primer (SEQ ID NO: 14) located in the transcription initiation sequence OsUbi2 of the transgene were designed. The PCR product was located in SEQ ID NO: 7 whose sequence is as shown in SEQ ID NO: 11.

Meanwhile, a PCR product was generated from the 3' terminus of the transgene insertion sequence; a primer (SEQ ID NO: 15) hybridized to the genomic DNA sequence flanking the 3' terminus of the transgene insertion sequence and a paired primer (SEQ ID NO: 16) located on the T-DNA were designed. The PCR product was located in SEQ ID NO: 8 whose sequence is as shown in SEQ ID NO: 12.

The junction sequence was a relatively short polynucleotide molecule, which was a novel DNA sequence that was diagnostic for DNA of the transgenic corn event QY2569-42 when detected in a polynucleic acid assay. The 5' junction sequences are listed in SEQ ID NO: 1, SEQ ID NO: 3 and SEQ ID NO: 5. The 3' junction sequences are listed in SEQ ID NO: 2, SEQ ID NO: 4 and SEQ ID NO: 6. A longer or shorter polynucleotide junction sequence may be selected from SEQ ID NO: 7 or SEQ ID NO: 8. The junction sequences (5' linking regions SEQ ID NO: 1, SEQ ID NO: 3 and SEQ ID NO: 5; 3' linking regions SEQ ID NO: 2, SEQ ID NO: 4 and SEQ ID NO: 6) were useful as DNA probes or DNA primer molecules in DNA detection methods.

The results in Figure 5 showed that using the genomic DNA of T2-T4 generation plants of QY2569-42 as templates, the specific PCR fragments of the exogenous inserted fragments 5' flanking and 3' flanking were all amplified from T2-T4 generations of QY2569-42, with sizes of 1068 bp and 798 bp, respectively. No bands were amplified from the transformation recipients and the vectors.

The DNA amplification conditions shown by the PCR detection results of the exogenous genes may be used in the above PCR conjugation assay to produce diagnostic amplicons for the transgenic corn event QY2569-42.

### 4. Localization of QY2569-42 insertion sequence in plant cells

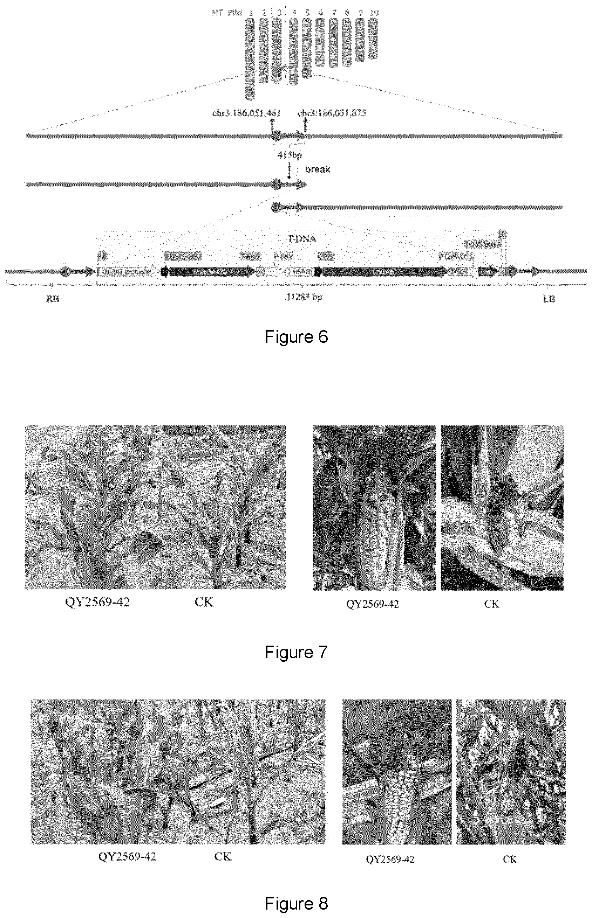

By Southern blotting, flanking sequence analysis and transformant-specific PCR detection, it was demonstrated that the insertion sequence of transgenic corn QY2569-42 had been integrated into the corn chromosome. The insertion of the T-DNA was a single copy insertion at a single site. Using the sequencing corn variety Mo17-CAU as a reference genome (https://download.maizegdb.org/Zm-Mo17-REFERENCE-CAU-1.0/Zm-Mo17-REFERENCE-CAU-1.0.fa.gz), the T-DNA insertion of QY2569-42 was localized on chromosome 3 (chr3: 186,051,461-186,051,875). During the T-DNA integration, 2 bp was randomly inserted between the LB end and the genome, and the 415-bp repeats at the 5' flanking sequence were integrated. The locating schematic of the insertion sequence in the genome is shown in Figure 6.

### Example 4 Assay of mVip3Aa20, Cry1Ab and PAT protein expression in transgenic corn event QY2569-42

The expression products of the insertion sequence in each organ and tissue were analyzed using the ELISA (enzyme-linked immunosorbent assay) method. The expressed proteins of the insertion sequence in the young leaves and seed tissues of QY2569-42 corns were determined. According to conventional planting methods, the sampling parts and time are: V2 (leaves, roots), V6 (leaves, stalks, roots), R1 (leaves, stalks, roots, pollens, tassels, filaments, female ears) and R6 (leaves, stalks, roots, grains). Using the non-transgenic corn ZM11 of the same type as a control, the expression levels of mVip3Aa20, Cry1Ab and PAT proteins in different tissues and organs of T2-T4 generations of QY2569-42 were determined.

The ELISA results of T2 to T4 generations of QY2569-42 showed that, mVip3Aa20, Cry1Ab and PAT proteins were detectable in all tissue samples of the transgenic corns. Each protein of interest had different contents per gram of different tissues, wherein in T4 generation, mVip3Aa20 protein had the highest content in leaves at R6 stage, which was 481.62 ± 88.35 µg/g fwt (i.e., content of protein of interest per gram of fresh tissues), and the lowest contents in roots at V2 and V6 stages, which were 36.74 ± 4.36, 36.19 ± 6.35 µg/g fwt, respectively; Cry1Ab protein had the highest content in leaves at R6 stage, which was 8.01 ± 2.55 µg/g fwt and the lowest content in pollens, which was 0.03 ± 0.01 µg/g fwt; PAT protein had the highest content in leaves at R6 stage, which was 6.17 ± 1.21 µg/g fwt and the lowest contents in pollens and grains, which were 0.63 ± 0.02 and 0.48 ± 0.08 µg/g fwt, respectively. However, no protein of interest was detected in any tissue of non-transgenic plants at various stages. The assay results showed that the expression of QY2569-42 exogenous proteins was stable.

### Example 5 Insect resistance examination of transgenic corn event QY2569-42

### 1. Indoor bioassay of corn plants QY2569-42

The test materials were planted in a greenhouse and 3-30 plants were planted for each material depending on the amounts of seeds. Conventional cultivation and management were employed and no insecticide applications during the whole growth period. The corresponding young parts were collected at 6-8 leaf stages, silking stage and filling stage and inoculated with *Ostrinia furnacalis, Spodoptera frugiperda* and *Helicoverpa armigera* indoors, respectively. *Mythimna separata* was fed with the corresponding young parts collected at 4-6 leaf stages. The resistance levels were evaluated.

When the corns grew to a proper stage, corn heart leaves, pistils and female ears were individually collected from the QY2569-42 corn field and the corresponding non-transgenic corn field to carry out insect resistance testing of in vitro tissues. Twenty samples were randomly collected from each area in each treatment. The leaves were cut and then an appropriate amount of which was placed in a plastic box, inoculated with 10 second instar larvae. Each box was 1 replicate and each treatment was repeated 10 times. Depending on the feeding status of the test materials by the larvae, the test materials of the same batch were replenished. The number of surviving larvae was counted and recorded. The experimental period was 5 days. The indoor bioassay results of 4 consecutive generations of QY2569-42 against *Ostrinia furnacalis, Mythimna separata, Spodoptera frugiperda* and *Helicoverpa armigera* showed that the leaves, filaments and grains of T3 and T4 generations of QY2569-42 had stable resistance against *Ostrinia furnacalis* and *Spodoptera frugiperda,* and except for a small number of survivors (survival rate less than 3%) in the T4 leaf stage, the survival rates of the rest were all 0; the resistance of the leaves against *Mythimna separata* was stable, wherein the survival rate in the T3 generation was 0 and the survival rate in the T4 generation was less than 3%; the resistance of the filaments and grains against *Helicoverpa armigera* was stable, wherein the survival rates in both T3 and T4 generations were 0. However, in the non-transgenic control and the insect-susceptible control, the survival rates of various target insects were all above 70%. It can be seen that the above plant tissues all maintained a high level of resistance, which was extremely distinct from the non-transgenic control and insect-susceptible control.

### 2. Resistance efficiency in field of QY2569-42 against target pests

Referring to relevant standards such as Chapter -10.1-2007 of Announcement No. 953 and NY/T 1248.5 of Ministry of Agriculture, DPS analysis software and Duncan's New MRT were employed to analyze and compare the differences of resistance against major lepidopteran pests *Ostrinia furnacalis, Mythimna separata, Spodoptera frugiperda* and *Helicoverpa armigera* between the transgenic corn QY2569-42 and the non-transgenic control, insect-susceptible control to determine the resistance levels of the transgenic corn QY2569-42 against the above pests.

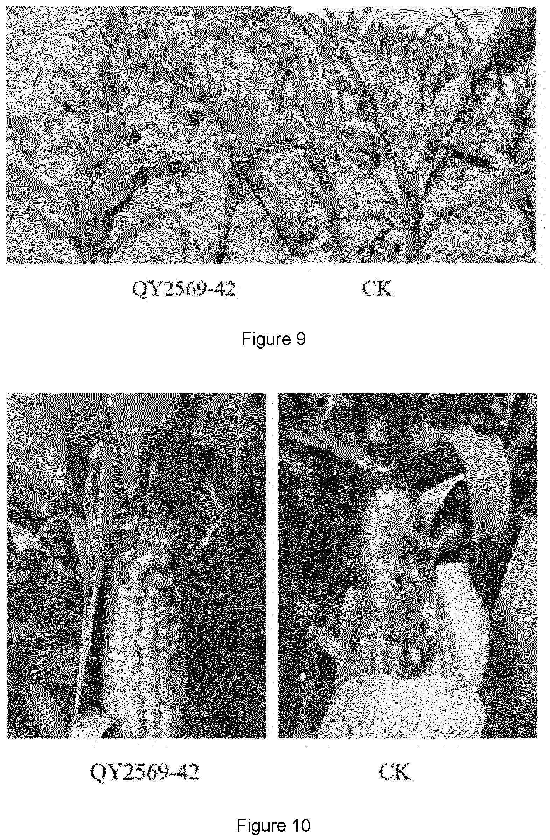

In pilot trials in Shandong and Hainan provinces, *Ostrinia furnacalis, Mythimna separata, Spodoptera frugiperda* and *Helicoverpa armigera* were manually inoculated in field and the resistance levels of T2-T4 generation plants of QY2569-42 against the target pests were tested. The results showed that QY2569-42 exhibited high resistance to *Ostrinia furnacalis, Mythimna separata, Spodoptera frugiperda* and *Helicoverpa armigera;* while the recipient corn ZM11 and insect-susceptible control exhibited high susceptibility or susceptibility. With the infestation of *Ostrinia furnacalis,* the plant height was inhibited which was distinctly different from QY2569-42. The specific effects of the field trials are shown in Figure 7-10.

### Example 6 Tolerance of transgenic corn event QY2569-42 against target herbicides

The evaluation of the tolerance of transgenic corn event QY2569-42 against target herbicides was carried out in accordance with "Evaluation of environmental impact of genetically modified plants and its derived products Herbicide-tolerant maize Part 1: Evaluation of the tolerance to herbicides" (Chapter-11.1-2007 of Announcement No. 953 of the Ministry of Agriculture). The target herbicide of the PAT protein expressed by QY2569-42 transformants was glufosinate. The corns were foliage-sprayed with glufosinate at 4-5 leaf stages. Plant heights and phytotoxicity rates were investigated and recorded 7 days, 14 days and 28 days after drug application, respectively. The results showed that there was no significant difference in emergence rate between the transgenic corn event QY2569-42 and the corresponding non-transgenic corn and the transformant QY2569-42 can tolerate a quadruple dose of glufosinate herbicide. The transformant QY2569-42 sprayed with a quadruple dose of glufosinate herbicide showed no significant difference in terms of seedling rate, phytotoxicity level and plant height observed 7 days, 14 days and 28 days after drug application, compared with the control without herbicide application and those applied with single or double doses. The non-transgenic recipient ZM11 had poorer tolerance to glufosinate, all dead 7 days after applying a single dose of glufosinate herbicide.

### Example 7 Observation and analysis of main agronomic traits of transgenic event QY2569-42

The growth stages and agronomic traits, including plant height, ear height, color of anthers, color of filaments, plant type, color of grains, color of cob, etc., of the transgenic event QY2569-42 and ZM11 (control) were investigated. The results showed that the transgenic corn was basically the same with the recipient variety in terms of agronomic traits, in which no apparent trait changes were found.

In summary, the transgenic corn event QY2569-42 has hereditary stability, good resistance against lepidopteran pests such as *Ostrinia furnacalis, Mythimna separata, Spodoptera frugiperda* and *Helicoverpa armigera,* and high tolerance to herbicides such as glufosinate at the same time, having no impact on yield. In addition, the detection method can accurately and rapidly identify whether a biological sample comprises DNA molecules of transgenic corn event QY2569-42.

The seeds corresponding to transgenic corn event QY2569-42 have been deposited in China Center for Type Culture Collection (abbr. CCTCC; add.: Wuhan University, No. 299 Bayi Road, Wuchang District, Wuhan City, Hubei Province 430072 P.R.C.) on August 22, 2023, with the classification name of Zea mays L. QY2569-42 and the accession number of CCTCC NO: P202336. The deposit will be deposited in CCTCC for 30 years.

Finally, it should be noted that the above embodiments are only to illustrate the technical solutions of the present invention and not to set limitations. Although the present invention has been described in detail with reference to preferred embodiments, it should be understood by one of ordinary skill in the art that the technical solutions of the present invention can be modified or equivalently replaced without departing from the spirit and scope of the technical solutions of the present invention.

## Claims

1. A nucleic acid sequence, wherein it is **characterized in that**, the nucleic acid sequence comprises:
(a) SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:10, SEQ ID NO:11 and/or SEQ ID NO:12; or
(b) a nucleic acid sequence complementary to (a);
the nucleic acid sequence is derived from transgenic corn event QY2569-42 and the transgenic corn event QY2569-42 is deposited in the form of seeds in China Center for Type Culture Collection with the accession number of CCTCC NO: P202336;
preferably, the nucleic acid sequence is an amplicon diagnostic for the presence of corn event QY2569-42.

2. A DNA construct comprising three expression cassettes, wherein,
a) the first expression cassette comprises in operable linkage an OsUbi2 promoter with a nucleic acid sequence as shown in SEQ ID NO: 29, a *mVip3Aa20* gene coding region with a nucleic acid sequence as shown in SEQ ID NO: 30, a chloroplast leading peptide CTP-TS-SSU with a nucleic acid sequence as shown in SEQ ID NO: 31, a T-Ara5 terminator with a nucleic acid sequence as shown in SEQ ID NO: 32 that terminates the expression of the gene;
b) the second expression cassette comprises in operable linkage a P-FMV promoter with a nucleic acid sequence as shown in SEQ ID NO: 33, a *cry1Ab* gene coding region with a nucleic acid sequence as shown in SEQ ID NO: 34, an I-HSP70 intron with a nucleic acid sequence as shown in SEQ ID NO: 35, a chloroplast localized peptide CTP2 with a nucleic acid sequence as shown in SEQ ID NO: 36, a T-Tr7 terminator with a nucleic acid sequence as shown in SEQ ID NO: 37 that terminates the expression of the gene;
c) the third expression cassette comprises in operable linkage a P-CaMV35S promoter with a nucleic acid sequence as shown in SEQ ID NO: 38, a *pat* gene coding region with a nucleic acid sequence as shown in SEQ ID NO: 39, a T-35S polyA termination sequence with a nucleic acid sequence as shown in SEQ ID NO: 40 that terminates the expression of the gene.

3. A DNA molecule having a sufficient length of contiguous nucleotides of SEQ ID NO: 10 to function as a DNA probe specific for SEQ ID NO: 10 in a sample of DNA derived from a corn plant, corn seed or corn cell, wherein the DNA probe comprises SEQ ID NO: 1 or SEQ ID NO: 2;
preferably, the probe is SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 11 or SEQ ID NO: 12.

4. A method of detecting the presence of DNA of transgenic corn event QY2569-42 in a sample, wherein it is **characterized in that**, it comprises:
contacting a sample to be tested with at least two primers in a nucleic acid amplification reaction;
performing a nucleic acid amplification reaction;
detecting the presence of an amplification product;
the amplification product comprises (a) SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8 and/or SEQ ID NO: 10; or (b) a nucleic acid sequence complementary to (a);
the nucleic acid sequence is derived from transgenic corn event QY2569-42 and the transgenic corn event QY2569-42 is deposited in the form of seeds in China Center for Type Culture Collection with the accession number of CCTCC NO: P202336;
preferably, the amplification product also comprises SEQ ID NO: 11 or a complementary sequence thereof, and/or SEQ ID NO: 12 or a complementary sequence thereof;
more preferably, the two primers comprise SEQ ID NO: 13 and SEQ ID NO: 14, or else SEQ ID NO: 15 and SEQ ID NO: 16.

5. A method of detecting the presence of DNA of transgenic corn event QY2569-42 in a sample, wherein it is **characterized in that**, it comprises
contacting a sample to be tested with a probe and the probe comprises SEQ ID NO: 1, SEQ ID NO: 2, SEQ ID NO: 3, SEQ ID NO: 4, SEQ ID NO: 5, SEQ ID NO: 6, SEQ ID NO: 7 or SEQ ID NO: 8 or a complementary sequence thereof;
hybridizing the sample to be tested with the probe under stringent hybridization conditions;
detecting the hybridization between the sample to be tested and the probe;
the transgenic corn event QY2569-42 is deposited in the form of seeds in China Center for Type Culture Collection with the accession number of CCTCC NO: P202336;
preferably, the probe also comprises SEQ ID NO: 11 or a complementary sequence thereof, or else SEQ ID NO: 12 or a complementary sequence thereof;
more preferably, at least one of the probes is labeled with at least one fluorophore.

6. A method of detecting the presence of DNA of transgenic corn event QY2569-42 in a sample, wherein it is **characterized in that**, it comprises
contacting a sample to be tested with a marker nucleic acid molecule and the marker nucleic acid molecule comprises SEQ ID NO: 1, SEQ ID NO: 2, SEQ ID NO: 3, SEQ ID NO: 4, SEQ ID NO: 5, SEQ ID NO: 6, SEQ ID NO: 7 or SEQ ID NO: 8 or a complementary sequence thereof;
hybridizing the sample to be tested with the marker nucleic acid molecule under stringent hybridization conditions;
detecting the hybridization between the sample to be tested and the marker nucleic acid molecule, and further determining the insect resistance and/or herbicide tolerance are genetically linked with the marker nucleic acid molecule by a marker assisted breeding analysis;
the transgenic corn event QY2569-42 is deposited in the form of seeds in China Center for Type Culture Collection with the accession number of CCTCC NO: P202336;
preferably, the marker nucleic acid molecule also comprises SEQ ID NO: 11 or a complementary sequence thereof, or else SEQ ID NO: 12 or a complementary sequence thereof.

7. A DNA detection kit, wherein it is **characterized in that**, it comprises at least one DNA molecule and the DNA molecule comprises SEQ ID NO: 1, SEQ ID NO: 2, SEQ ID NO: 3, SEQ ID NO: 4, SEQ ID NO: 5, SEQ ID NO: 6, SEQ ID NO: 7 or SEQ ID NO: 8 or a complementary sequence thereof, which may function as one of DNA primers or a probe specific for transgenic corn event QY2569-42 or progeny thereof;
the transgenic corn event QY2569-42 is deposited in the form of seeds in China Center for Type Culture Collection with the accession number of CCTCC NO: P202336;
preferably, when functioning as a probe, the DNA molecule also comprises SEQ ID NO: 11 or a complementary sequence thereof, or else SEQ ID NO: 12 or a complementary sequence thereof.

8. A method for protecting a corn plant from insect infestation, wherein it is **characterized in that**, it comprises providing at least one transgenic corn plant cell in the diet of a target insect, wherein the transgenic corn plant cell sequentially comprises SEQ ID NO: 1, the nucleic acid sequence of position 777-11945 of SEQ ID NO: 10 and SEQ ID NO: 2, or, SEQ ID NO: 3, the nucleic acid sequence of position 777-11945 of SEQ ID NO: 10 and SEQ ID NO: 4, or, SEQ ID NO: 5, the nucleic acid sequence of position 777-11945 of SEQ ID NO: 10 and SEQ ID NO: 6 in its genome, or else the genome of the transgenic corn plant cell comprises SEQ ID NO: 10; the target insect that feeds on the transgenic corn plant cell is inhibited from further ingesting the corn plant.

9. A method for protecting a corn plant from damage caused by herbicides, wherein it is **characterized in that**, it comprises applying a herbicide containing an effective amount of glufosinate to a field growing at least one transgenic corn plant, wherein the transgenic corn plant sequentially comprises SEQ ID NO: 1, the nucleic acid sequence of position 777-11945 of SEQ ID NO: 10 and SEQ ID NO: 2, or, SEQ ID NO: 3, the nucleic acid sequence of position 777-11945 of SEQ ID NO: 10 and SEQ ID NO: 4, or, SEQ ID NO: 5, the nucleic acid sequence of position 777-11945 of SEQ ID NO: 10 and SEQ ID NO: 6 in its genome, or else the genome of the transgenic corn plant comprises SEQ ID NO: 10; the transgenic corn plant has tolerance to glufosinate herbicide.

10. A method of controlling weeds in a field growing corn plants, wherein it is **characterized in that**, it comprises applying a herbicide containing an effective amount of glufosinate to a field growing at least one transgenic corn plant, wherein the transgenic corn plant sequentially comprises SEQ ID NO: 1, the nucleic acid sequence of position 777-11945 of SEQ ID NO: 10 and SEQ ID NO: 2, or, SEQ ID NO: 3, the nucleic acid sequence of position 777-11945 of SEQ ID NO: 10 and SEQ ID NO: 4, or, SEQ ID NO: 5, the nucleic acid sequence of position 777-11945 of SEQ ID NO: 10 and SEQ ID NO: 6 in its genome, or else the genome of the transgenic corn plant comprises SEQ ID NO: 10; the transgenic corn plant has tolerance to glufosinate herbicide.

11. A method of cultivating an insect-resistant corn plant, wherein it is **characterized in that**, it comprises planting at least one corn seed, wherein the genome of the corn seed comprises the nucleic acid sequence of a specific region and the nucleic acid sequence of the specific region sequentially comprises SEQ ID NO: 1, the nucleic acid sequence of position 777-11945 of SEQ ID NO: 10 and SEQ ID NO: 2, or, SEQ ID NO: 3, the nucleic acid sequence of position 777-11945 of SEQ ID NO: 10 and SEQ ID NO: 4, or, SEQ ID NO: 5, the nucleic acid sequence of position 777-11945 of SEQ ID NO: 10 and SEQ ID NO: 6, or else the nucleic acid sequence of the specific region comprises SEQ ID NO: 10; allowing the corn seed to grow into a corn plant;
infesting the corn plant with a target insect to harvest a plant having reduced plant damage compared with other plants that do not possess the nucleic acid sequence of the specific region.

12. A method of cultivating a glufosinate-tolerant corn plant, wherein it is **characterized in that**, it comprises planting at least one corn seed, wherein the genome of the corn seed comprises the nucleic acid sequence of a specific region and the nucleic acid sequence of the specific region sequentially comprises SEQ ID NO: 1, the nucleic acid sequence of position 777-11945 of SEQ ID NO: 10 and SEQ ID NO: 2, or, SEQ ID NO: 3, the nucleic acid sequence of position 777-11945 of SEQ ID NO: 10 and SEQ ID NO: 4, or, SEQ ID NO: 5, the nucleic acid sequence of position 777-11945 of SEQ ID NO: 10 and SEQ ID NO: 6, or else the nucleic acid sequence of the specific region comprises SEQ ID NO: 10;
allowing the corn seed to grow into a corn plant;
spraying the corn plant with an effective amount of glufosinate to harvest a plant having reduced plant damage compared with other plants that do not possess the nucleic acid sequence of the specific region.

13. A method of cultivating an insect-resistant and glufosinate-tolerant corn plant, wherein it is **characterized in that**, it comprises planting at least one corn seed, wherein the genome of the corn seed comprises the nucleic acid sequence of a specific region and the nucleic acid sequence of the specific region sequence sequentially comprises SEQ ID NO: 1, the nucleic acid sequence of position 777-11945 of SEQ ID NO: 10 and SEQ ID NO: 2, or, SEQ ID NO: 3, the nucleic acid sequence of position 777-11945 of SEQ ID NO: 10 and SEQ ID NO: 4, or, SEQ ID NO: 5, the nucleic acid sequence of position 777-11945 of SEQ ID NO: 10 and SEQ ID NO: 6, or else the nucleic acid sequence of the specific region comprises SEQ ID NO: 10;
allowing the corn seed to grow into a corn plant;
spraying the corn plant with an effective amount of glufosinate to harvest a plant having reduced plant damage compared with other plants that do not possess the nucleic acid sequence of the specific region, wherein the plant having reduced plant damage is also resistant to feeding damage by insects.

14. A method of producing an insect-resistant corn plant, wherein it is **characterized in that**, it comprises hybridizing a corn plant, whose genome sequentially comprises SEQ ID NO: 1, the nucleic acid sequence of position 777-11945 of SEQ ID NO: 10 and SEQ ID NO: 2, or, SEQ ID NO: 3, the nucleic acid sequence of position 777-11945 of SEQ ID NO: 10 and SEQ ID NO: 4, or, SEQ ID NO: 5, the nucleic acid sequence of position 777-11945 of SEQ ID NO: 10 and SEQ ID NO: 6, with another corn plant, thereby producing a large number of progeny plants; selecting a progeny plant whose genome comprises the nucleic acid sequence of a specific region, wherein the nucleic acid sequence of the specific region sequentially comprises SEQ ID NO: 1, the nucleic acid sequence of position 777-11945 of SEQ ID NO: 10 and SEQ ID NO: 2, or, SEQ ID NO: 3, the nucleic acid sequence of position 777-11945 of SEQ ID NO: 10 and SEQ ID NO: 4, or, SEQ ID NO: 5, the nucleic acid sequence of position 777-11945 of SEQ ID NO: 10 and SEQ ID NO: 6, or else the nucleic acid sequence of the specific region comprises SEQ ID NO: 10 and the progeny plant has reduced plant damage caused by insect feeding;
preferably, the method comprises sexually crossing a first parental corn plant of transgenic corn event QY2569-42 that is insect-resistant with a second parental corn plant that lacks insect resistance, thereby producing a large number of progeny plants;
infesting the progeny plants with a target insect;
selecting the progeny plant having reduced plant damage compared with other plants that do not possess the nucleic acid sequence of the specific region;
the transgenic corn event QY2569-42 is deposited in the form of seeds in China Center for Type Culture Collection with the accession number of CCTCC NO: P202336.

15. A method of producing a glufosinate-tolerant corn plant, wherein it is **characterized in that**,
it comprises hybridizing a corn plant, whose genome sequentially comprises SEQ ID NO: 1, the nucleic acid sequence of position 777-11945 of SEQ ID NO: 10 and SEQ ID NO: 2, or, SEQ ID NO: 3, the nucleic acid sequence of position 777-11945 of SEQ ID NO: 10 and SEQ ID NO: 4, or, SEQ ID NO: 5, the nucleic acid sequence of position 777-11945 of SEQ ID NO: 10 and SEQ ID NO: 6, with another corn plant, thereby producing a large number of progeny plants; selecting a progeny plant whose genome comprises the nucleic acid sequence of a specific region, wherein the nucleic acid sequence of the specific region sequentially comprises SEQ ID NO: 1, the nucleic acid sequence of position 777-11945 of SEQ ID NO: 10 and SEQ ID NO: 2, or, SEQ ID NO: 3, the nucleic acid sequence of position 777-11945 of SEQ ID NO: 10 and SEQ ID NO: 4, or, SEQ ID NO: 5, the nucleic acid sequence of position 777-11945 of SEQ ID NO: 10 and SEQ ID NO: 6, or else the nucleic acid sequence of the specific region comprises SEQ ID NO: 10 and the progeny plant has glufosinate tolerance;
preferably, the method comprises sexually crossing a first parental corn plant of transgenic corn event QY2569-42 that is glufosinate-tolerant with a second parental corn plant that lacks glufosinate tolerance, thereby producing a large number of progeny plants;
treating the progeny plants with glufosinate herbicide;
selecting the progeny plant that is glufosinate-tolerant;
the transgenic corn event QY2569-42 is deposited in the form of seeds in China Center for Type Culture Collection with the accession number of CCTCC NO: P202336.

16. A method of producing a corn plant that is resistant to insects and tolerant to glufosinate herbicide application, wherein it is **characterized in that**, it comprises sexually crossing a first parental corn plant of transgenic corn event QY2569-42 that is glufosinate-tolerant and insect-resistant with a second parental corn plant that lacks glufosinate tolerance and/or insect resistance, thereby producing a large number of progeny plants;
treating the progeny plants with glufosinate;
selecting the progeny plant that is glufosinate-tolerant, wherein the progeny plant that is glufosinate-tolerant is also resistant to feeding damage by insects;
the transgenic corn event QY2569-42 is deposited in the form of seeds in China Center for Type Culture Collection with the accession number of CCTCC NO: P202336.

17. A method for improving tolerance of a corn plant, comprising:
a) constructing the DNA construct according to claim 3;
b) inserting the DNA construct into the genome of a corn cell;
c) regenerating the corn cell into a corn plant; and
d) selecting a corn plant comprising the DNA construct;
preferably, improvement of tolerance of a corn plant includes resistance to an effective amount of at least one herbicide and the herbicide is preferably glufosinate; and/or
improvement of tolerance of a corn plant includes resistance to at least one insect and the insect is preferably *Ostrinia furnacalis, Mythimna separata, Spodoptera frugiperda* and/or *Helicoverpa armigera.*

18. A composition produced from transgenic corn event QY2569-42, wherein it is **characterized in that**, the composition is corn flour, corn meal, corn oil, corn silk or corn starch; the transgenic corn event QY2569-42 is deposited in the form of seeds in China Center for Type Culture Collection with the accession number of CCTCC NO: P202336.

19. An agricultural product or commodity produced from transgenic corn event QY2569-42, wherein it is **characterized in that**, the agricultural product or commodity is corn flour, corn meal, corn oil, corn starch, corn gluten, corn cake, cosmetics or filling agents; the transgenic corn event QY2569-42 is deposited in the form of seeds in China Center for Type Culture Collection with the accession number of CCTCC NO: P202336.

20. A plant cell, plant part, plant, seed or non-living plant material, comprising the nucleic acid sequence according to claim 1.
